Europäisches Patentamt

European Patent Office

Office européen des brevets

(11) Veröffentlichungsnummer : **0 307 449 B1**

## (12) EUROPÄISCHE PATENTSCHRIFT

(45) Veröffentlichungstag der Patentschrift :
26.08.92 Patentblatt 92/35

(21) Anmeldenummer : 88903222.3

(22) Anmeldetag : 29.03.88

(86) Internationale Anmeldenummer :
PCT/EP88/00261

(87) Internationale Veröffentlichungsnummer :
WO 88/07710 06.10.88 Gazette 88/22

(51) Int. Cl.$^5$ : **G05B 19/417, C12Q 1/04, G01N 35/00, G01N 33/02**

(54) VERFAHREN ZUR MIKROBIOLOGISCHEN UNTERSUCHUNG VON PROBEN.

(30) Priorität : 31.03.87 DE 3710663
23.06.87 DE 3720733

(43) Veröffentlichungstag der Anmeldung :
22.03.89 Patentblatt 89/12

(45) Bekanntmachung des Hinweises auf die
Patenterteilung :
26.08.92 Patentblatt 92/35

(84) Benannte Vertragsstaaten :
AT BE CH DE FR GB IT LI LU NL SE

(56) Entgegenhaltungen :
EP-A- 0 240 134
DE-A- 3 430 170
FR-A- 2 424 961
US-A- 4 287 301
International Laboratory, volume 12, No 7,
September 1982, (Fairfiel, Connecticut, US),
G.L. Hawk et al.: "A robotic approachto automated sample preparation", pages 48-56 see
pages 48-51

(73) Patentinhaber : **Lufthansa Service GmbH
Flughafen Frankfurt-Ost
W-6000 Frankfurt 75 (DE)**

(72) Erfinder : **GORK, Fritz-Peter
Kriemheldring 11a
W-6234 Hattersheim 2 (DE)**
Erfinder : **MÜLLER, Klaus-Dieter
Am Gartenweg 9
W-6086 Riedstadt 6 (DE)**
Erfinder : **SCHWEITZER, Doris
Am Hühnerberg 25
W-6238 Wallau (DE)**

(74) Vertreter : **Lieck, Hans-Peter, Dipl.-Ing.
Feddersen Laule Scherzberg Undritz
Widenmayerstrasse 36
W-8000 München 22 (DE)**

## Beschreibung

Die Erfindung betrifft ein Verfahren zur mikrobiologischen Untersuchung von Proben, insbesondere Nahrungsmittel-Proben, nach dem Oberbegriff des Anspruches 1.

Bisher wurde dieses Verfahren, wie jede mikrobiologische Untersuchung, ausschließlich von Hand durchgeführt. Dies ist vertretbar, wenn es um wenige Proben geht. In der Nahrungsmittelindustrie müssen jedoch Proben häufig und in großer Anzahl auf die Keimzahl untersucht werden. Erschwerend kommt hinzu, daß bei mikrobiologischen Untersuchungen steril gearbeitet werden muß und keine allzu große Zeit von der Anlieferung der Proben bis zur Einstellung in den Brutschrank vergehen darf, weil sonst aufgrund der Vermehrung der Keime schon bei Zimmertemperatur des Untersuchungserbebnis verfälscht würde.

Der Erfindung liegt die Aufgabe zugrunde, das Untersuchungs-Verfahren der eingangs genannten Art so auszugestalten, daß es nach dem bereitstellen der Proben bis zum Einstellen der geimpften Nährböden in den Brutschrank automatisch ablaufen kann.

Diese Aufgabe ist erfindungsgemäß ausgehend von dem eingangs genannten Verfahren zur mikrobiologischen Untersuchung von Proben dadurch gelöst, daß das Zerkleinern und Homogenisieren des Beutelinhaltes durch ein als Stomacher ausgebildetes Laborgerät erfolgt, bei welchem der zugeklemmte Plastikbeutel zwischen einer feststehenden Backe und zwei alternierend oszillierenden Backen gepreßt wird, daß das Impfen des Nährbodens durch ein als Spiralplater ausgebildetes Laborgerät erfolgt, bei welchem die Probenflüssigkeit spiralförmig auf den Nährboden ausgeimpft wird, daß sämtliche Handhabungsvorgänge mittels einer mechanischen Hand eines Labor-Roboters eines Labor-Roboter-Systems durchgeführt werden, und daß der Roboter und alle beteiligten Laborgeräte durch eine entsprechend dem Untersuchungsablauf programmierte Kontrolleinheit gesteuert werden. Vorteilhafte Ausgestaltungen dieses Verfahrens gehen aus den Unteransprüchen hervor.

Wesentliches Merkmal des erfindungsgemäßen Verfahrens ist die Verwendung eines Labor-Roboter-Systems mit einem Handhabungs-Roboter und einer programmierbaren Kontrolleinheit, welche den Roboter und die in seinem Bewegungsbereich angeordneten Laborgeräte steuert, mit welchen der Roboter zusammenwirkt. Ein solches System ist zwar als universelles System für die Durchführung verschiedener Laboroperationen, insbesondere auch bei der Bearbeitung von Proben, bereits bekannt (Labor-Roboter-System der Firma Zymark Corporation, Hopkinton, Massachusetts, USA, beschrieben in: International Laboratory, Band 12, Nr. 7, September 1982, (Fairfield, Conneticut, US), G.L. Hawk et al.: "A robotic approach to automated sample preparation", Seiten 48-56). Es ist dieses jedoch bisher nie für die Durchführung der relativ komplexen mikrobiologischen Untersuchungen eingerichtet und benutzt worden. Der erfindungsgemäß angestrebte Erfolg wird erst dadurch erreicht, daß das Labor-Roboter-System in Kombination mit zwei ganz bestimmten, für sich ebenfalls bereits bekannten weiteren Laborgeräten eingesetzt wird, nämlich dem sogenannten Stomacher und dem sogenannten Spiralplater. Der Stomacher erlaubt die Zerkleinerung und Homogenisierung von Proben, die in Plastikbeuteln enthalten sind, bei geschlossenem Plastikbeutel. Dadurch wird es möglich, die zu untersuchenden Proben während der gesamten Untersuchung in Plastikbeuteln zu belassen. Dies wiederum ist Voraussetzung dafür, daß die Proben individuell vom Labor-Roboter gehandhabt werden können, ohne daß hierbei Proben oder Probenreste miteinander in Berührung kommen, wodurch das Untersuchungsergebnis verfälscht würde. Die mikrobiologische Untersuchung verlangt weiter, daß von jeder Probe Probenflüssigkeit mit unterschiedlicher Konzentration auf den Nährboden ausgeimpft wird. Der Spiralplater, bei welchem die Probenflüssigkeit auf den sich drehenden Nährboden durch eine in radialer Richtung bewegte Düse, also spiralförmig und damit in einem Arbeitsgang in unterschiedlicher Konzentration ausgeimpft wird, schafft die Voraussetzung dafür, daß auch dieser Vorgang durch das Labor-Roboter-System bewältigt werden kann, was bei einem mehrstufigen Ausimpfen, z.B. mit unterschiedlich konzentrierten Probenflüssigkeiten wegen der zu hohen Komplexität eines solchen Vorganges und des zu großen Zeitaufwandes kaum möglich wäre. Insgesamt wird mit dem erfindungsgemäßen Verfahren erreicht, daß insbesondere Nahrungsmittel-Proben in großer Anzahl vollautomatisch untersucht werden können. Es ist lediglich notwendig, die z.b. im Lebensmittelbetrieb entnommenen Proben in dem Plastikbeutel bereitzustellen. Anschließend werden sie nach dem erfindungsgemäßen Verfahren bis zum Einstellen der fertig beimpften, in Petrischalen befindlichen Nährböden in den Brutschrank vollautomatisch bearbeitet.

Bei der Ausgestaltung des neuen Verfahrens nach Anspruch 2 ist die bisher geübte genaue Zumessung der Proben, die mit dem Roboter nur schwierig durchzuführen wäre, nicht notwendig. Es können die im Gewicht nur ungefähr abgeschätzten Proben, so wie sie von der Entnahme kommen, verarbeitet werden. Das gewünschte bestimmte Gewichtsverhältnis zwischen Probe und physiologischer Nährlösung wird durch entsprechende Zumessung der Nährlösung hergestellt, die vorzugsweise als zeitabhängige, aber auch als volumetrische Zumessung leicht automatisch durchzuführen ist. Hierbei übernimmt die Kontrolleinheit die Auswertung der Wägung der Probe und die Berechnung der notwendigen Menge an Nährlösung.

Die Ausgestaltung des neuen Verfahrens nach dem Anspruch 3 ermöglicht die weitere Beschleunigung desselben durch Verschachtelung von Verfahrensabschnitten, indem der Stomacher sehr bald für eine neue Probe freigemacht wird. Außerdem ist die saubere Entfernung der Plastikbeutel, deren Inhalt ausgewertet ist, durch einfaches Fallenlassen in die Abfallöffnung gewährleistet.

In gleicher Richtung wirkt die Weiterbildung gemäß Anspruch 4. Das die Pipette umgebende mechanische Sieb wirkt als Filter, durch das nur Probenflüssigkeit hindurch gelangen kann. Natürlich muß die Pipette nach jedem Entnahmevorgang gereinigt und sorgfältig sterilisiert werden. Durch die Anregung des Desinfektions- und Reinigungsbades mit Ultraschall wird gleichzeitig eine mechanische Reinigung insbesondere des Siebes der Pipette erreicht, wodurch das Auswechseln eines Filters nach jedem Entnahmevorgang eingespart wird. Zur Zeitersparnis kann man die Saug-Pipette abtropfen lassen, während der Stomacher arbeitet.

Der an sich bekannte Spiralplater besitzt eine eingebaute Dosierpumpe. bei der üblichen Verwendung im Rahmen von Hand durchgeführter Untersuchungen wird die auszuimpfende Probenflüssigkeit mittels der rückwärts laufenden Dosierpumpe durch die Ausimpf-Düse des Spiralplaters eingesaugt. Normalerweise werden mit der Probenflüssigkeit von einer Probe zwei Nährböden spiralförmig beimpft. Dies bedeutete bisher zwei Ansaugvorgänge. bei der Ausgestaltung des neuen Verfahrens nach Anspruch 5 wird Probenflüssigkeit für den Spiralplater nur einmal angesaugt und es ist trotzdem die Beimpfung mehrerer Nährböden möglich, was der automatischen Durchführung des Verfahrens weiter entgegen kommt. Zur sicheren gegenseitigen Trennung der Proben kann der Spiralplater jeweils nach dem Ausimpfen der Probenflüssigkeit einer Probe mit Reinigungs- und Desinfektionsflüssigkeiten durchspült werden. Die Spülung wird ebenfalls, gesteuert durch die Kontrolleinheit, automatisch durchgeführt.

Wesentliche Voraussetzung für den automatischen Untersuchungsablauf ist eine sichere Handhabung der gefüllten Plastikbeutel durch den Labor-Roboter. Diese ist dann besonders gut gewährleistet, wenn der Labor-Roboter für die Handhabung der Plastikbeutel eine Hand gemäß Anspruch 6 benutzt. Beim Transport wird der Plastikbeutel zwischen den Klemmbacken eingeklemmt. Zum Öffnen des Beutels werden die Öffnungen in den Klemmbacken mit Unterdruck beaufschlagt und die Klemmbacken auseinander bewegt. Die Öffnüngen können auch im geschlossenen Zustand der Klemmbacken mit Unterdruck beaufschlagt bleiben. Dann werden die Plastikbeutel besonders sicher festgehalten.

Im Zuge einer Untersuchung muß jeder Plastikbeutel mehrmals transportiert und dabei am Ziel, z.b. auf der Waage oder im Stomacher oder in der Halteeinrichtung genau positioniert abgestellt werden. Damit dies unbeeinträchtigt durch Eigenbewegungen des Beutels genau gelingt, hat sich die Vorgehensweise nach Anspruch 7 als vorteilhaft herausgestellt. Durch das Öffnen des Plastikbeutels während des Zwischenhaltes wird der Plastikbeutel stabilisiert und Pendelbewegungen des Beutels hören auf.

Gewöhnlich werden bei mikrobiologischen Untersuchungen mit der Probenflüssigkeit zusätzlich spezielle Nährböden beimpft, jedoch nur in einer festen Konzentration. Zur Einsparung an Nährböden ist es üblich, jeden Nährboden - selbstverständlich an verschiedenen Stellen, z.b. Sektoren desselben - mehrmals mit verschiedenen Proben zu beimpfen. Diese Beimpfung kann im Rahmen des erfindungsgemäßen Verfahrens mit Vorteil gemäß dem Anspruch 8 geschehen.

Die Petrischalen mit frischem Nährboden, und zwar sowohl diejenigen für die spiralige Beimpfung als auch diejenigen für die Mehrfach-Beimpfung, werden zweckmäßigerweise gemäß Anspruch 9 für die automatische Handhabung bereit gehalten. Hierbei findet der Labor-Roboter die einzelne Petrischale immer an der gleichen Stelle, was für einen möglichst einfachen automatischen Verfahrensablauf wichtig ist. Die Unterbringung im Stapel ist im übrigen platzsparend. Außerdem können die Stapelmagazine während des Untersuchungsablaufs ohne Störung desselben nachgefüllt werden.

Da an allen Stellen peinlich steril gearbeitet werden muß, sind die Petrischalen in der üblichen Weise mit Deckeln verschlossen. Diese Deckel werden am besten gemäß Anspruch 10 gehandhabt. Es hat sich als schwierig herausgestellt, das Entfernen und Wiederaufsetzen der Deckel auch dem Labor-Roboter selber zu überlassen.

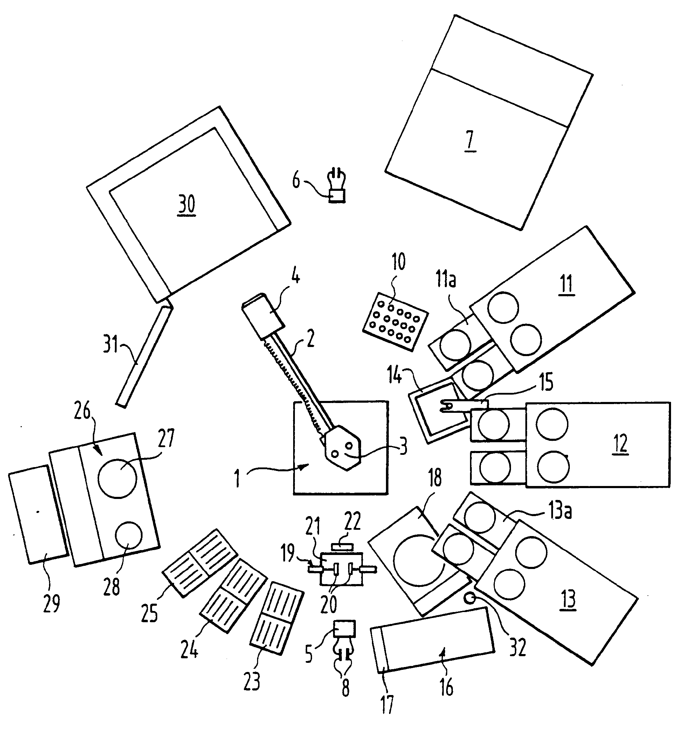

Im folgenden ist die Erfindung mit weiteren vorteilhaften Einzelheiten anhand eines schematisch dargestellten Ausführungsbeispiels erläutert. Die einzige Figur zeigt in schematischer Draufsicht eine Labor-Anordnung zur automatischen mikrobiologischen Untersuchung von Nahrungsmittel-Proben.

Die gesamte, in der einzigen Figur dargestellte Untersuchungs-Anordnung befindet sich auf einer großen Tischplatte und umfaßt mehrere einzelne Geräte. Im Zentrum der Anordnung befindet sich ein Labor-Roboter 1. Dieser umfaßt einen horizontalen Arm 2, der von einer senkrechten Säule 3 wegsteht. Der Arm ist an der Säule 3 in der Höhe verstellbar und durch Drehen der Säule 3 um ihre senkrechte Mittelachse in horizontaler Richtung über einen Kreisbogen von etwas mehr als 360° in beiden Richtungen verschwenkbar. Außerdem kann der Arm 2 gegenüber der Säule 3 in seiner Längsrichtung verschoben werden, so daß sein freies Ende unterschiedliche Abstände von der Säule 3 einnimmt. Die Figur zeigt den Arm 2 in einer teilweise ausgefahrenen Stellung. Am freien Ende weist der Arm 2 eine Antriebseinheit 4 auf, mit welcher verschiedene, jeweils in mehreren Freiheitsgraden bewegbare Roboterhände durch einen Steckvorgang koppelbar sind. beim erfindungsgemäßen Verfahren wird mit zwei verschiedenen, noch näher zu erläuternden Roboterhänden 5 und 6 gearbeitet.

Der Labor-Roboter 1 gehört zu einem Labor-Roboter-System, welches außerdem eine Kontrolleinheit 7 auf Mikroprozessor-Basis mit einer Tastatur und einem Bildschirm (nicht näher dargestellt) umfaßt. Die Kontrolleinheit ist durch den Benutzer programmierbar und steuert entsprechend dem Programm den Labor-Roboter 1 sowie alle weiteren, an der Untersuchung beteiligten Laborgeräte.

Die an der Untersuchung beteiligten Laborgeräte sind im Kreis um den Labor-Roboter herum angeordnet, so daß sie von der Roboterhand erreicht werden können. Zu den Laborgeräten gehört ein Lochmagazin 10 mit mehreren, in einem Rechteckraster angeordneten senkrechten Löchern, die vor Beginn einer Untersuchungsreihe jeweils mit einer Einmal-Saugpipette bestückt werden. Die Einmal-Saugpipetten können vom Roboter 1 mittels der Hand 6 einzeln aus dem Lochmagazin 10 entnommen werden. Die Kontrolleinheit 7 ist mittels eines Unterprogramms so programmierbar, daß sie sich das Loch, aus dem jeweils zuletzt eine Saug-Pipette entnommen wurde, merkt und die Roboterhand bei der nächsten Entnahme zum jeweils nächsten, noch gefüllten Loch führt.

Im Uhrzeigersinn auf das Lochmagazin 10 folgen drei gleichartige Stapelmagazine 11, 12 und 13 für die Aufnahme von Petrischalen, in welchen sich Nährböden befinden. Jedes Stapelmagazin enthält die mit Deckeln verschlossenen Petrischalen in zwei Stapeln. Jedem Stapel ist am unteren Ende ein Schieber, z.b. 11a, zugeordnet, welcher bei Betätigung, gesteuert durch die Kontrolleinheit 7, die jeweils unterste Petrischale des Stapels in den Aktionsbereich des Roboters 1 vorschiebt. Die Magazine 11 und 12 enthalten Petrischalen, deren Nährboden durch Stege im Boden der Petrischalen in vier Sektoren unterteilt ist. Sie sind für das sogenannte Drop-plating bestimmt, bei welchem jeder Sektor des Nährbodens mit einem Tropfen Probenflüssigkeit von einer jeweils anderen Probe geimpft wird. Das Magazin 13 enthält normale Petrischalen ohne Stege und einem entsprechend durchgehenden Nährboden, der für das sogenannte Spiral-plating bestimmt ist, bei welchem Probenflüssigkeit spiralförmig auf den Nährboden ausgeimpft wird.

Zwischen und vor den beiden Stapelmagazinen 11 und 12 befindet sich ein offener Behälter 14 mit einem Desinfektions- und Reinigungsbad, das durch Ultraschall angeregt ist. Neben dem Behälter 14 ist eine noch zu erläuternde Abtropf-Halterung 15 angeordnet.

In Umfangsrichtung folgt auf das Stapelmagazin 13 ein sogenannter Stomacher 16. Hierbei handelt es sich um ein an sich bekanntes Gerät zum Zerkleinern und Homogenisieren von Proben, die sich in Plastikbeuteln befinden. Am linken Ende besitzt der Stomacher 16 einen Schacht 17, der durch eine Klappe verschließbar ist. Die Plastikbeutel werden von oben in den geöffneten Schacht eingestellt, wo sie nach dem selbsttätigen, mittels eines Motors oder eines Hydraulikzylinders bewirkten Schließen der Schachtklappe zwischen einer feststehenden backe und zwei nebeneinander liegenden, alternierend hin- und herbewegten backen gepreßt werden.

Vor dem Stomacher 16 befindet sich eine elektronische Waage 18. In Umfangsrichtung folgt auf den Stomacher 16 eine nicht näher dargestellte Halterung für die eine Hand 5 des Roboters. Die Hand 5 ist für die Handhabung von mit Proben gefüllten Plastikbeuteln bestimmt. Sie weist dazu zwei Klemmbacken 8 auf, zwischen denen die oben offenen Plastikbeutel an ihrem oberen Rand ergriffen und eingeklemmt werden können. In den Klemmbacken 8 sind in nicht näher gezeigter Weise mit Unterdruck beaufschlagbare Öffnungen vorgesehen. Wenn diese Öffnungen mit Unterdruck beaufschlagt werden, bleiben die Beutelwände an der jeweiligen Backe haften, so daß die Beutel durch Auseinanderbewegen der Klemmbacken 8 geöffnet werden können und trotzdem nicht aus der Klemmbacke herausfallen.

Zwischen der Halterung für die Hand 5 und dem Roboter 1 befindet sich eine ortsfeste Haltestation 19 für die Kunststoffbeutel, die analog zur Hand 5 zwei Klemmbacken 20 jeweils mit Unterdruck-Öffnungen aufweist. Unter der Halterung 19 befindet sich in der Tischplatte eine Abfallöffnung 21. In radialer Richtung unmittelbar neben der stationären Haltestation 19 am Rand der Abfallöffnung 21 ist ein Abstreifer 22 fest montiert.

In Umfangsrichtung auf die Halterung für die Hand 5 folgen drei gleichartige, jeweils radial ausgerichtete Beutelmagazine 23, 24 und 25. Drei Beutelmagazine sind lediglich beispielhaft angegeben. In der Praxis können insbesondere auch mehr vorhanden sein. bei der in der praktischen Erprobung befindlichen Anordnung waren zuletzt fünf Beutelmagazine vorgesehen. Jedes Beutelmagazin besitzt mehrere, nach oben offene, in radialer Richtung längliche Öffnungen, in die mit Nahrungsmittel-Proben gefüllte Plastikbeutel eingestellt werden können. In Umfangsrichtung haben die länglichen Öffnungen einen solchen Abstand voneinander, daß die Plastikbeutel mittels der Hand 5 bzw. deren Klemmbacken 8 am Oberrand einzeln ergriffen und aus dem Magazin herausgehoben werden können. Wie bei dem Lochmagazin 10 sorgt ein Unterprogramm dafür, daß die Kontrolleinheit 7 dem Roboter 1 bzw. dessen Hand nacheinander zur jeweils nächsten Magazinöffnung führt.

An die drei Beutelmagazine schließt sich in Umfangsrichtung ein sogenannter Spiralplater 26 an. Dies ist ein an sich bekanntes Gerät mit einem drehbaren Aufnahmeteller 27 für Petrischalen. Über dem Aufnahmeteller 27 befindet sich in hier nicht näher gezeigter Weise eine in Bezug auf den Aufnahmeteller 27 in radialer Richtung bewegbare Impfdüse, aus der mittels einer Dosierpumpe auszuimpfende Probenflüssigkeit ausgespritzt wird. Durch gleichzeitige Drehung des Aufnahmetellers 27 und radiale Bewegung der Impfdüse wird die Probenflüssigkeit in einer spiraligen Bahn auf den Nährboden in der Petrischale ausgeimpft, und zwar mit einer kontinuierlich sich ändernden Ausimpf-Konzentration, die von der jeweiligen Relativgeschwindigkeit zwischen Düse und Nährboden sowie gegebenenfalls von einem entsprechenden Steuerungsprofil der Dosierpumpe abhängt. Der Spiralplater hat außerdem eine feststehende Zwischenablage 28 für die Petrischale, an welcher mittels einer nicht näher gezeigten Saug- und Hebeeinrichtung deren Deckel abgehoben werden kann. Außerhalb des Spiralplaters 26 befindet sich ein wie alle anderen Laborgeräte an die Kontrolleinheit 7 angeschlossenes Steueraggregat 29. Dieses bewirkt über entsprechende Schlauchverbindungen und hydraulische Ventile einerseits das "Laden" des Spiralplaters mit Probenflüssigkeit, welche hierzu durch eine noch zu erläuternde Sieb-Pipette angesaugt wird, und andererseits das Durchspülen des Spiralplaters und der Sieb-Pipette mit Wasser und Desinfektionsflüssigkeit nach jedem abgeschlossenen Impfvorgang.

In Umfangsrichtung neben dem Spiralplater ist ein großer Brutschrank 30 mit einer Türe 31 aufgestellt, in welchen die Petrischalen nach dem Beimpfen des Nährbodens vom Roboter 1 in z. B. sechs Stapeln eingestellt werden, wobei sich die Kontrolleinheit 7 mittels eines Unterprogramms wiederum analog zu der Entnahme aus dem Lochmagazin 10 die Position der jeweils obersten Petrischale im Stapel merkt.

Schließlich ist neben dem Brutschrank eine Halterung für die zweite Hand 6 des Roboters 1 vorgesehen. Es handelt sich hierbei um eine Universal-Hand mit Greifern, die für die Handhabung der Petrischalen und der Einmal-Pipetten geeignet sind. Gesteuert durch die Kontrolleinheit 7 kann der Roboter die beiden Hände 5 und 6 wahlweise mit der Halterung 4 koppeln, wobei die jeweils unbenutzte Hand auf der ihr zugeordneten Halterung abgelegt bleibt, bis sie wieder benötigt wird.

Zur Durchführung einer mikrobiologischen Untersuchungsreihe werden z.b. in einem Lebensmittelbetrieb dem Gewicht nach nur grob geschätzte Nahrungsmittel-Proben entnommen und in die Plastikbeutel eingefüllt. Die mit den Proben gefüllten Plastikbeutel werden in die entsprechenden Öffnungen der Beutelmagazine 23 bis 25 von Hand eingestellt. Jedes Magazin hat z.B. acht Öffnungen, so daß bei z.B. fünf Magazinen bis zu vierzig Plastikbeutel eingestellt werden können. Außerdem werden die Stapelmagazine 11, 12 und 13 mit Petrischalen befüllt, in denen sich die für die Untersuchung benötigten Nährböden befinden. Gesteuert durch die entsprechend programmierte Kontrolleinheit 7 werden nun die Proben vollautomatisch nacheinander bearbeitet, und zwar in folgendem Verfahrensablauf:

Der Roboter 1 nimmt die Hand 5 auf und bewegt diese zum ersten Plastikbeutel im Magazin 23. Dieser wird zwischen den Klemmbacken 8 ergriffen, aus dem Magazin herausgehoben, zur Waage 18 transportiert und auf deren Waagschale durch Öffnen der Klemmbacken 8 abgestellt. beim Öffnen der Klemmbacken wird die Hand außerdem in vertikaler Richtung bewegt, um sicher zu stellen, daß der Plastikbeutel nicht versehentlich an den Klemmbacken hängen bleibt. Die Kontrolleinheit 7 fragt das Gewicht des gefüllten Plastikbeutels ab und ermittelt hieraus durch Subtraktion des bekannten Beutelgewichtes das Probengewicht. Der Beutel wird nun wieder von der Hand 8 ergriffen, unter einen neben der Waage 18 vorgesehenen Auslaufstutzen 32 für Nährmittellösung gebracht und dort geöffnet. Der Beutel wird nun mit Nährmittellösung befüllt. Hierbei wird eine bestimmte Menge Nährmittellösung, entsprechend dem ermittelten Probengewicht, von der Kontrolleinheit 7 zugemessen, so daß Probe und Nährmittellösung in einem gewünschten Gewichtsverhältnis stehen. Zum Zumessen wird eine zeitgesteuerte Pumpe verwendet.

Anschließend wird der Beutel wieder geschlossen und in der geöffneten Schacht 17 des Stomachers 16 eingestellt. Der Beutel bleibt noch von der Hand 5 gehalten, während der Schacht 17 mittels der Schachtklappe motorisch geschlossen wird. Am Ende der Schließbewegung klemmt die Schachtplatte den Beutel fest. Die Hand 5 entfernt sich und der Stomacher beginnt in der erläuterten Weise zu arbeiten.

In dem Desinfektions- und Reinigungsbad im Behälter 14 ist die bereits erwähnte Sieb-Pipette, d.h. eine von einem feinen mechanischen Sieb umschlossene Pipette abgestellt, die über einen Schlauch an das Steueraggregat 29 angeschlossen ist. Der Roboter 1 fährt zur Sieb-Pipette, hebt sie aus dem Bad und stellt sie auf der Abtropf-Halterung 15 ab, wo die anhaftende Desinfektionslösung abtropfen kann. Anschließend kehrt er zum Stomacher zurück, ergreift dort den Plastikbeutel im mittlerweile wieder geöffneten Schacht 17 und überführt den Plastikbeutel in die stationäre Haltestation 19. Auf dem Wege dorthin legt der Roboter 1 einen Zwischenhalt ein, bei welchem der Plastikbeutel geöffnet und dadurch zur Ruhe gebracht wird, was das anschließende genaue Positionieren zwischen den Klemmbacken 20 der stationären Haltestation 19 erleichtert. Nachdem sich die Roboter-Hand 5 entfernt hat, wird der Plastikbeutel nunmehr in der Haltestation 19 durch Auseinanderbewegen der Klemmbacken 20 geöffnet. In der Zwischenzeit holt der Roboter die Sieb-Pipette von der Abtropf-Halterung 15 und führt sie nach kurzem Eintauchen in ein nicht gezeigtes Wässerungsbad in den geöffneten Plastikbeutel ein. Über das Steueraggregat 29 wird nunmehr eine gewisse Menge der in dem Plastikbeutel enthaltenen Probenflüssigkeit unter Zurückhaltung der festen Bestandteile der Probe in einen Schlauchabschnitt im Steueraggregat 29 eingesaugt. Anschließend wird die Sieb-Pipette wieder aus dem Plastikbeutel herausgezogen und zurück in das Desinfektions- und Reinigungsbad im Behälter 14 transportiert. Dann wechselt der Roboter 1 die Hand 5 gegen die Hand 6 aus. Mit der Hand 6 entnimmt er eine Einmal-Pipette dem Lochmagazin 10 und anschließend - unter Festhalten der Einmal-Pipette, eine Petrischale dem Stapelmagazin 13. Diese Petrischale führt er auf die Zwischenablage 28 des Spiralplaters 26, wo der Deckel der Petrischale abgehoben wird, und von dort auf den Aufnahmeteller 27. Dort wird der Nährboden der Petrischale beimpft, wozu die in dem Schlauchabschnitt enthaltene Probenflüssigkeit mit Wasserdruck in die Dosierpumpe des Spiralplaters hineingedrückt und mittels der Dosierpumpe durch die Impfdüse in der beschriebenen Weise ausgespritzt wird. Während der Impfvorgang läuft, taucht der Roboter 1 die Einmal-Pipette in den geöffneten Plastikbeutel in der Haltestation 19 und saugt mit dieser weitere Probenflüssigkeit auf. Anschließend bewegt sich der Roboter zurück zum Spiralplater, ergreift dort die Petrischale, führt sie auf die Zwischenablage 28, wo der Deckel wieder aufgelegt wird, und bringt sie von dort in den Brutschrank 30 auf einen der drei Stapel, wobei die ganze Zeit die gefüllte Einmal-Pipette mitwandert. Sodann wird eine zweite Petrischale aus dem Stapelmagazin 13 entnommen, in der beschriebenen Weise zum Spiralplater transportiert, dort geöffnet und auf den Aufnahmeteller 27 gestellt.

Während die zweite Petrischale beimpft wird, bewegt sich der Roboter zu einem der beiden Stapelmagazine 11 oder 12. Dort werden mittels einer ortsfesten Hebe- und Saugeinrichtung die Deckel von zwei Petrischalen abgehoben und jeweils ein neuer Sektor ihres Nährbodens aus der Einmal-Pipette tropfenweise beimpft. Anschließend werden die Deckel wieder aufgelegt. Dann fährt der Roboter mit der Hand 6 zu dem Abstreifer 22, mittels welchem die Einmal-Pipette durch Hochziehen der Hand abgestreift wird, so daß sie in die Abfallöffnung 21 fällt. Anschließend wird inzwischen geimpfte zweite Petrischale vom Spiralplater 26 zum Brutschrank 30 gebracht. Schließlich wird der Plastikbeutel von den Klemmbacken 20 der Haltestation 19 freigegeben und fällt ebenfalls in die Abfallöffnung 21. Die Hand 6 wird wieder gegen die Hand 5 ausgetauscht und ein neuer Arbeitszyklus kann beginnen. Durch eine Hin- und Herbewegung der Schieber des Magazins 13 werden zwei neue Petrischalen in den Aktionsbereich des Roboters 1 gebracht. Wenn am Ende eines Zyklus alle vier Sektoren der Petrischalen aus den Magazinen 11 und 12 beimpft sind, werden vor dem Handwechsel in einem eingefügten Arbeitsgang die entsprechenden Petrischalen ebenfalls in den Brutschrank 30 überstellt und neue Petrischalen durch Hin- und Herbewegung der Schieber der Magazine 11 und 12 in den Aktionsbereich des Roboters 1 gebracht.

Bei einer Alternative des erläuterten Verfahrensablaufes bleiben die Plastikbeutel nach dem Zerkleinern und Homogenisieren der Probe zunächst im Stomacher, so daß die Probenflüssigkeit dort aus dem geöffneten Beutel entnommen wird. Am Schluß wird der Plastikbeutel direkt in die Abfallöffnung 21 eingeworfen. Dadurch ist der Verfahrensablauf etwas einfacher. Andererseits ist die Gefahr einer unerwünschten Verunreinigung des Stomachers etwas größer.

**Patentansprüche**

1. Verfahren zur mikrobiologischen Untersuchung von Proben, insbesondere Nahrungsmittel-Proben, bei welchem jede Probe in einem Plastikbeutel angeliefert wird, der Probe im Plastikbeutel physiologische Nährlösung in einem bestimmten Gewichtsverhältnis zugesetzt wird, der Beutelinhalt auf mechanischem Wege zerkleinert und homogenisiert wird, eine kleine Teilmenge des Beutelinhaltes mittels einer Pipette über ein Filter als Probenflüssigkeit entnommen wird, die Probenflüssigkeit auf mindestens einen in einer Petrischale befindlichen Nährboden ausgeimpft wird und die beimpften Petrischalen in einen Brutschrank eingestellt werden,
dadurch gekennzeichnet,
daß das Zerkleinern und Homogenisieren des Beutelinhaltes durch ein als Stomacher (16) ausgebildetes Laborgerät erfolgt, bei welchem der zugeklemmte Plastikbeutel zwischen einer feststehenden Backe und zwei alternierend oszillierenden Backen gepreßt wird,
daß das Impfen des Nährbodens durch ein als Spiralplater (26) ausgebildetes Laborgerät erfolgt, bei welchem die Probenflüssigkeit spiralförmig auf den Nährboden ausgeimpft wird,
daß sämtliche Handhabungsvorgänge mittels einer mechanischen Hand (5; 6) eines Labor-Roboters (1) eines Labor-Roboter-Systems (1, 7) durchgeführt werden, und daß der Roboter (1) und alle beteiligten Laborgeräte durch eine entsprechend dem Untersuchungsablauf programmierte Kontrolleinheit (7) gesteuert werden.

2. Verfahren nach Anspruch 1,
dadurch gekennzeichnet,

daß die Probe im Plastikbeutel gewogen und die physiologische Nährlösung jeweils in einer dem ermittelten Gewicht entsprechenden Menge zugesetzt wird.

3. Verfahren nach Anspruch 1 oder 2,
dadurch gekennzeichnet,
daß der bearbeitete Plastikbeutel aus dem Stomacher (16) zu einer stationären Halteeinrichtung (19) transportiert wird, wo die Probenflüssigkeit entnommen wird, wobei sich die stationäre Halteeinrichtung vorzugsweise über einer Abfallöffnung (21) befindet.

4. Verfahren nach einem der Ansprüche 1 bis 3,
dadurch gekennzeichnet,
daß zum Entnehmen von Probenflüssigkeit eine von einem mechanischen Sieb Pipette verwendet wird, die nach jedem Entnahmevorgang in ein Desinfektions- und Reinigungsbad (14) eingestellt wird, das durch Ultraschall angeregt ist, und daß die Sieb-Pipette vor der nächsten Probennahme aus dem Desinfektions- und Reinigungsbad entnommen und zum Abtropfen abgestellt wird, während der Stomacher (16) arbeitet.

5. Verfahren nach einem der Ansprüche 1 bis 4,
dadurch gekennzeichnet,
daß die Probenflüssigkeit aus dem Plastikbeutel in einen Schlauchabschnitt eingesaugt und aus diesem durch Wasserdruck in die Dosierpumpe des Spiralplaters (26) transferiert wird.

6. Verfahren nach einem der Ansprüche 1 bis 5,
dadurch gekennzeichnet,
daß der Labor-Roboter (1) für die Handhabung der Plastikbeutel eine Hand (5) mit zwei Klemmbacken benutzt, in welchen mit Unterdruck beaufschlagbaren Öffnungen für das Öffnen der Plastikbeutel vorgesehen sind.

7. Verfahren nach Anspruch 6,
dadurch gekennzeichnet,
daß beim Transport der Plastikbeutel zur Beruhigung der Beutelbewegung ein Zwischenhalt eingelegt und während desselben der Plastikbeutel geöffnet wird.

8. Verfahren nach einem der Ansprüche 1 bis 7,
dadurch gekennzeichnet,
daß mittels einer Einmal-Saugpipette zusätzliche Probenflüssigkeit aus dem Plastikbeutel entnommen und auf mindestens einen Nährboden in einer Petrischale ausgeimpft wird, welcher Impfungen mehrerer Proben aufnimmt, wobei die Eimnal-Saugpipetten vom Labor-Roboter (1) jeweils aus einem Lochmagazin (10) entnommen und nach dem Ausimpfen über der Abfallöffnung (21) von der Roboterhand (6) abgestreift werden.

9. Verfahren nach einem der Ansprüche 1 bis 8,
dadurch gekennzeichnet,
daß die Petrischalen mit dem frischen Nährboden in Stapelmagazinen (11; 12; 13) bereitgehalten werden, aus denen sie mittels eines Schiebers (11a; 12a; 13a) jeweils einzeln in den Aktionsbereich des Labor-Roboters (1) vorgeschoben werden.

10. Verfahren nach Anspruch 8 oder 9,
dadurch gekennzeichnet,
daß die Petrischalen mit Deckeln verschlossen sind, die mittels Saughebern am Spiralplater (26) und/oder an den Stapelmagazinen (11; 12; 13) abgehoben und wieder aufgesetzt werden.

## Claims

1. A method for the microbiological examination of samples, in particular food samples, in which each sample is supplied in a plastic bag, physiological nutrient solution in a particular weight ratio is added to the sample in the plastic bag, the contents of the bag are comminuted mechanically and are homogenised, a small partial quantity of the contents of the bag is removed by means of a pipette via a filter as sample liquid, the sample liquid is injected out onto at least one nutrient base lying in a petri dish and the injected petri dishes are placed into an incubator, characterised in that

the comminution and homogenisation of the contents of the bag takes place by a laboratory instrument constructed as a stomacher (16), in which the plastic bag, which is clamped shut, is pressed between a fixed jaw and two alternatingly oscillating jaws, that the injecting of the nutrient base takes place by means of a laboratory instrument constructed as a spiral plater (26), in which the sample liquid is injected in a spiral form onto the nutrient base,

that all the handling processes are carried out by means of a mechanical hand (5; 6) of a laboratory robot (1) of a laboratory robot system (1, 7),

and that the robot (1) and all the laboratory instruments involved are controlled by a control unit (7) which is programmed in accordance with the sequence of examination.

2. A method according to Claim 1,
characterised in that
the sample in the plastic bag is weighed and the physiological nutrient solution is added in an amount corresponding to the determined weight.

3. A method according to Claim 1 or 2,
characterised in that
the processed plastic bag is conveyed from the stomacher (16) to a stationary holding device (19), where the sample liquid is removed, in which the stationary holding device is preferably situated over a waste opening (21).

4. A method according to one of Claims 1 to 3,
characterised in that
for the removal of sample liquid, a pipette is used, surrounded by a mechanical screen, which pipette is placed into a disinfecting- and cleaning bath (14) after each removal procedure, which bath is activated by ultrasonics, and that the screen pipette is removed from the disinfecting- and cleaning bath before the next sampling and is set to drip, whilst the stomacher (16) operates.

5. A method according to one of Claims 1 to 4,
characterised in that
the sample liquid is sucked out of the plastic bag into a tube section and is transferred therefrom by water pressure into the dosing pump of the spiral plater (26).

6. A method according to one of Claims 1 to 6,
characterised in that
the laboratory robot (1) for handling the plastic bags uses a hand (5) with two clamping jaws, in which openings, able to be acted upon by negative pressure, are provided for opening the plastic bags.

7. A method according to Claim 6,
characterised in that
in the conveying of the plastic bags, to stabilize the movement of the bags, an intermediate support is fitted and during same the plastic bag is opened.

8. A method according to one of Claims 1 to 7,
characterised in that
by means of a disposable suction pipette, additional sample liquid is removed from the plastic bag and injected onto at least one nutrient base in a petri dish, which receives injections of several samples, in which the disposable suction pipettes are in each case removed by the laboratory robot (1) from a hole magazine (10) and after injecting out are stripped off over the waste opening (21) by the robot hand (6).

9. A method according to one of Claims 1 to 8,
characterised in that
the petri dishes with the fresh nutrient base are kept ready in stacking magazines (11; 12; 13), from which they are advanced, by means of a slider (11a; 12a; 13a) in each case individually into the range of action of the laboratory robot (1).

10. A method according to Claim 8 or 9,

characterised in that
the petri dishes are closed with lids, which are raised by means of suction lifters on the spiral plater (26) and/or on the stacking magazines (11; 12; 13) and placed in position again.

## Revendications

1. Procédé d'analyse microbiologique d'échantillons, en particulier d'échantillons de produits alimentaires, dans lequel chaque échantillon est fourni dans un sachet de matière plastique, la solution nutritive physiologique est ajoutée à l'échantillon dans le sachet de matière plastique dans un rapport pondéral déterminé, le contenu du sachet est fragmenté et homogénéisé par voie mécanique, une faible quantité partielle du contenu du sachet est prélevée à l'aide d'une pipette via un filtre à titre de liquide d'échantillonnage, le liquide d'échantillonnage est inoculé à au moins un milieu nutritif disposé dans une boîte de Pétri et les boîtes de Pétri inoculées sont placées dans un incubateur, caractérisé en ce qu'on procède à la fragmentation et à l'homogénéisation du contenu des sachets par un appareil de laboratoire de type digesteur (Stomacher) (16) dans lequel le sachet de matière plastique bloqué est pressé entre un mors fixe et deux mors à oscillation alternée, on effectue l'inoculation du milieu nutritif par un appareil de laboratoire de type inoculateur à spirale (Spiralplater) (26) dans lequel le liquide d'échantillonnage est inoculé en spirale au milieu nutritif, on réalise toutes les manipulations via une pince mécanique (5, 6) d'un robot de laboratoire (1) d'un système robotisé de laboratoire (1, 7) et le robot (1) et tous les appareils de laboratoire intéressés sont commandés par une unité de contrôle (7) programmée conformément au déroulement de l'analyse.

2. Procédé selon la revendication 1, caractérisé en ce que l'échantillon est pesé dans le sachet de matière plastique et la solution physiologique nutritive est ajoutée respectivement en quantité correspondant au poids obtenu.

3. Procédé selon la revendication 1 ou 2, caractérisé en ce que le sachet de matière plastique traité est transporté du digesteur (16) à un dispositif d'arrêt fixe (19) où le liquide d'échantillonnage est prélevé, le dispositif d'arrêt fixe (19) se trouvant de préférence au-dessus d'une ouverture d'évacuation (21).

4. Procédé selon l'une quelconque des revendications 1 à 3, caractérisé en ce que, pour prélever le liquide d'échantillonnage, on utilise une pipette entourée d'un tamis mécanique qui est introduite, après chaque opération de prélèvement, dans un bain de désinfection et de nettoyage (14) qui est activé par des ultrasons et la pipette-tamis est retirée du bain de désinfection et de nettoyage avant la prise d'échantillon suivante et déposée à égoutter tandis que le digesteur (16) travaille.

5. Procédé selon l'une quelconque des revendications 1 à 4, caractérisé en ce que le liquide d'échantillonnage est aspiré du sachet de matière plastique dans une section de tube flexible d'où il est transféré par pression hydraulique dans la pompe de dosage du inoculateur à spirale (26).

6. Procédé selon l'une quelconque des revendications 1 à 5, caractérisé en ce que le robot de laboratoire (1) utilise pour la manipulation du sachet de matière plastique une pince (5) avec deux mors de serrage dans lesquels sont prévues des ouvertures sous dépression pour ouvrir le sachet de matière plastique.

7. Procédé selon la revendication 6, caractérisé en ce que, pour transporter le sachet de matière plastique jusqu'à l'arrêt du mouvement du sachet, on intercale un arrêt intermédiaire au cours duquel le sachet de matière plastique est ouvert.

8. Procédé selon l'une quelconque des revendications 1 à 7, caractérisé en ce qu'on prélève via une pipette d'aspiration jetable une quantité supplémentaire de liquide d'échantillonnage du sachet de matière plastique et on l'inocule à au moins un milieu nutritif dans une boîte de Pétri, qui reçoit des inoculations de plusieurs échantillons, les pipettes d'aspiration jetables étant prélevées respectivement dans un magasin à orifices (10) par le robot de laboratoire (1) et étant extraites de la pince (6) du robot au-dessus d'une orifice d'évacuation (21) après l'inoculation.

9. Procédé selon l'une quelconque des revendications 1 à 8, caractérisé en ce que les boîtes de Pétri sont préparées avec le milieu nutritif frais dans les magasins d'empilage (11, 12, 13), à partir desquels elles sont poussées en avant individuellement à portée d'action du robot de laboratoire (1) via un coulisseau (11a, 12a, 13a).

10. Procédé selon la revendication 8 ou 9, caractérisé en ce que les boîtes de Pétri sont fermées par des couvercles qui sont soulevés et remis en place par des dispositifs de levage installés sur le inoculateur à spirale (26) et/ou sur les magasins d'empilage (11, 12, 13).